# EUROPEAN PATENT SPECIFICATION

(11) **EP 0 730 906 B1**
(45) Date of publication and mention of the grant of the patent: **11.08.1999**
(21) Application number: 96103586.2
(22) Date of filing: 07.03.1996
(51) Int. Cl.: B01J 23/44, B01J 23/42, B01J 23/40, B01J 23/62, C07C 5/333, C07C 15/46

(54) **Method for selective oxidation of hydrogen, and method for dehydrogenation of hydrocarbon**
Verfahren für die selektive Oxidation von Wasserstoff und für die Dehydrierung von Kohlenwasserstoffen
Méthode d'oxydation sélective de l'hydrogène et méthode de déshydrogénations des hydrocarbures

(30) Priority: 08.03.1995 JP 4874095; 28.03.1995 JP 6985595; 16.05.1995 JP 11705395; 16.05.1995 JP 11705495; 16.05.1995 JP 11705595
(43) Date of publication of application: 11.09.1996
(73) Proprietor: Mitsubishi Chemical Corporation, Chiyoda-ku Tokyo (JP)
(72) Inventor: Tomoatsu, Iwakura, c/o Tsukuba Research Center, Inashiki-gun, Ibaraki 300-03 (JP); Makoto, Takiguchi, c/o Tsukuba Research Center, Inashiki-gun, Ibaraki 300-03 (JP)
(74) Representative: TER MEER STEINMEISTER & PARTNER GbR

(56) References cited:
- EP-A- 0 089 183
- EP-A- 0 336 622
- EP-A- 0 501 265
- EP-A- 0 505 863
- US-A- 4 435 607
- US-A- 4 479 902

## Description

The present invention relates to a method for selectively oxidizing hydrogen present in a gas mixture formed at the time of dehydrogenation of a hydrocarbon to produce a dehydrogenated hydrocarbon.

A process for producing a dehydrogenated hydrocarbon by dehydrogenation of a hydrocarbon, has been disclosed in many literatures. For example, a process for preparing styrene by dehydrogenation of methylbenzene is industrially practiced by means of an iron-type catalyst. However, a dehydrogenation reaction is usually subject to a restriction of equilibrium, whereby it is difficult to attain a good yield. Further, the dehydrogenation reaction is an endothermic reaction, and if the reaction is carried out by an insulated reactor, the reaction temperature decreases as the reaction proceeds, whereby it is difficult to obtain the desired product in good yield.

Under these circumstances, some methods have already been proposed. For example, UK Patent No. 1,404,641 discloses a process and a catalyst for selectively oxidizing hydrogen in a gas mixture comprising unreacted methylbenzene, styrene and hydrogen after the dehydrogenation of ethylbenzene. This method is effective for the preparation of styrene, but A-type zeolite or alumina having platinum supported thereon is used as a catalyst for selective oxidation of hydrogen, and its performance is not necessarily satisfactory.

Also USP 4,565,898 discloses a method of using a catalyst having e.g. platinum, tin and lithium supported on alumina for a similar process. However, this catalyst is supported also on alumina, and its performance is not fully satisfactory.

Further, Japanese Unexamined Patent Publications No. 89945/1983 and No. 298678/1994 disclose a method for selectively oxidizing hydrogen in a gas mixture comprising styrene, ethylbenzene and hydrogen, formed by the dehydrogenation reaction of ethylbenzene, by means of a catalyst containing tin oxide, or tin oxide and an alkali metal. This catalyst is noteworthy as a catalyst employing no platinum, but its performance is not necessarily adequate.

As described above, conventional catalysts are not satisfactory in their performance as catalysts for selectively oxidizing hydrogen in a gas mixture comprising an unreacted hydrocarbon, a hydrogenated hydrocarbon and hydrogen, formed by the dehydrogenation reaction of a hydrocarbon.

The present inventors have conducted extensive studies to solve the above problems and as a result, have found that selective oxidation of hydrogen can be carried out highly efficiently with a catalyst comprising a component having platinum and/or palladium supported on a certain specific oxide carrier. The present invention has been accomplished on the basis of this discovery.

A process for catalytically dehydrogenating a saturated hydrocarbon at the contact of tin oxide impregnated with Pt or Pd is disclosed in EP-A-0 336 622. In this prior art reading, the carrier is claimed beforehand at 350°C.

The present invention provides a method for selective oxidation of hydrogen, which comprises selectively oxidizing hydrogen in a gas mixture containing a hydrocarbon and hydrogen by contacting the gas mixture with an oxygen-containing gas in the presence of an oxidation catalyst, wherein a catalyst comprising a component having platinum and/or palladium supported on at least one carrier calcined at a temperature of from 800 to 1500°C and selected from the group consisting of tin oxide, titanium oxide, tantalum oxide and niobium oxide, is used as the oxidation catalyst.

Further, the present invention provides a method for dehydrogenation of a hydrocarbon, which comprises selectively oxidizing hydrogen in a gas mixture which is obtained by subjecting a feed hydrocarbon to a dehydrogenation reaction in the presence of a dehydrogenation catalyst and which comprises a dehydrogenated hydrocarbon, an unreacted feed hydrocarbon and hydrogen, by contacting the gas mixture with an oxygen-containing gas in the presence of an oxidation catalyst, and further subjecting a hydrocarbon-containing gas obtained by the oxidation reaction to a dehydrogenation reaction, wherein a catalyst comprising a component having platinum and/or palladium supported on at least one carrier calcined at a temperature of from 800 to 1500°C and selected from the group consisting of tin oxide, titanium oxide, tantalum oxide and niobium oxide, is used as the oxidation catalyst.

Now, the present invention will be described in detail with reference to the preferred embodiments.

The catalyst for selective oxidation of hydrogen to be used in the method of the present invention is a catalyst containing tin oxide, titanium oxide, tantalum oxide or niobium oxide having platinum and/or palladium supported thereon.

The oxide carrier to be used in the present invention, can be prepared by a suitable method which is commonly employed for the preparation of catalysts. For example, an aqueous alkaline solution such as aqueous ammonia, an alkali metal carbonate solution or an alkali metal hydrogencarbonate solution may be added to an aqueous solution of a salt of tin, titanium, tantalum or niobium with stirring, or water may be added to a solution of an organic salt of tin, titanium, tantalum or niobium with stirring, to form a precipitate of a hydroxide, which is then collected by filtration and washed. This hydroxide is dried and then calcined at a proper temperature to convert it into an oxide at a temperature of from 800 to 1,500°C. The oxide thus prepared may be tabletted or extrusion-molded as the case requires. The starting material salt of tin, titanium, tantalum or niobium is not particularly limited, and a chloride, a nitrate, a sulfate, an organic salt or a hydroxide of such a metal may be employed. Further, such a salt may directly be calcined to obtain a catalyst carrier in the form of an oxide.

As a method for supporting platinum and/or palladium on such an oxide carrier, a method may, for example, be mentioned in which an aqueous solution of a salt of platinum or palladium is impregnated to the oxide after calcination, followed by drying and calcining at a temperature of from 50 to 1,000°C. The starting material salt of platinum or palladium is not particularly limited, and a halide, a hydroxide, a sulfate or an organic salt thereof may, for example, be employed.

The amount of platinum or palladium supported, is usually from 0.01 to 10 wt%, preferably from 0.05 to 5 wt%, based on the oxide carrier. If the amount supported is too small, the catalytic activity for the oxidation reaction tends to be low. On the other hand, even if the amount supported is increased beyond the above range, no substantial further effects to the reaction will be obtained, and such will be disadvantageous from the viewpoint of the costs.

The oxidation catalyst of the present invention is useful for a reaction for selectively oxidizing hydrogen in a gas mixture containing hydrogen and a hydrocarbon by contacting the gas mixture with an oxygen-containing gas. Such a reaction is conducted preferably within a temperature range of from 300 to 800°C, more preferably from 400 to 700°C. If the temperature is too high, the selectivity for hydrogen decreases, and combustion of the hydrocarbon increases, such being undesirable. If the temperature is too low, the activity tends to be low, although the selectivity may not substantially be affected.

A specific example of the gas mixture containing hydrogen and a hydrocarbon may be a gas mixture which is obtained by subjecting a feed hydrocarbon to a dehydrogenation reaction in the presence of a dehydrogenation catalyst and which comprises a dehydrogenated hydrocarbon, an unreacted feed hydrocarbon and hydrogen.

The oxygen-containing gas may, for example, be a gas containing from 1 to 100% of molecular oxygen. Specifically, air, oxygen-enriched air or air diluted with an inert gas may suitably be employed. Further, steam may be incorporated into the oxgen-containing gas.

A typical process to which the selective oxidation catalyst and the selective oxidation method of the present invention may be applied, is as follows.

In a first reaction zone, a dehydrogenation reaction of a feed hydrocarbon is carried out by a dehydrogenation catalyst, and then, a gas mixture containing a dehydrogenated hydrocarbon, an unreacted feed hydrocarbon and hydrogen, discharged from this first reaction zone, will be sent to a second reaction zone. In this second reaction zone, selective oxidation of hydrogen is carried out in the presence of the selective oxidation catalyst of the present invention by means of an oxygen-containing gas introduced anew, whereby the temperature once lowered by the first dehydrogenation reaction as an endothermic reaction will be raised, and the restriction by equilibrium of the dehydrogenation reaction will be eliminated by the consumption of hydrogen. Further, the gas discharged from this second reaction zone will be sent to a third dehydrogenation reaction zone which is similar to the first reaction zone, and dehydrogenation of an unreacted hydrocarbon will be carried out. As the temperature required for the reaction has already been recovered and the restriction by equilibrium has already been eliminated in the second reaction zone, a higher yield can be attained in the third dehydrogenation reaction zone.

If necessary, the reaction can be carried out by further adding a combination of the above selective oxidation reaction zone and the dehydrogenation reaction zone.

It is common to conduct a dehydrogenation reaction in the presence of steam. Also in the above reaction process, steam may be present.

As a typical specific example of the above dehydrogenation process, a dehydrogenation process of ethylbenzene may, for example, be mentioned. Namely, for example, a gas mixture of ethylbenzene and steam is sent to a first reaction zone, where an iron-type catalyst comprising iron and an alkali metal as the main active components, is present, and a dehydrogenation reaction is carried out at a temperature within a range of from 500 to 800°C under a pressure within a range of from 0.05 to 10 atm. Then, a gas mixture of an unreacted ethylbenzene, formed styrene, hydrogen and steam, will be sent to a second reaction zone. In the second reaction zone, selective oxidation of hydrogen is carried out in the presence of the oxidation catalyst of the present invention by means of an oxygen-containing gas introduced anew. Then, this reaction gas is sent to a third reaction zone, where dehydrogenation of unreacted ethylbenzene is carried out again by an iron-type catalyst, to obtain styrene in good yield.

As mentioned above, according to the method of the present invention, the restriction by equilibrium will be removed, and the decrease of the reaction temperature can be supplemented, whereby it is possible to obtain styrene in a yield substantially higher as compared with conventional dehydrogenation reactions.

Now, the present invention will be described in further detail with reference to Examples. However, it should be understood that the present invention is by no means restricted to such specific Examples.

### EXAMPLE 1

### Preparation of a catalyst

3N aqueous ammonia was gradually added to an aqueous stannous chloride solution with stirring to form a precipitate of a hydroxide. When the pH exceeded 7, addition of aqueous ammonia was stopped. Then, the formed precipitate of tin hydroxide was collected by filtration and washed with water. The obtained precipitate of tin hydroxide was dried in a drier at 120°C overnight. The dried precipitate was calcined in a muffle furnace at 600°C for 2 hours. A small amount of water was added to this calcined product, followed by extrusion molding to obtain pellets having an average size of 3 mm in diameter × 10 mm, which were again calcined at 1,000°C for 3 hours. The calcined product was further pulverized to obtain tin oxide of a particle size of from 0.85 to 1.0 mm. 5.2 g of the tin oxide of from 10 to 20 mesh was immersed in 2 g of an aqueous solution of chloroplatinic acid (H₂PtCl₆·6H₂O) containing 0.0021 g of platinum and then dried by a rotary evaporator under reduced pressure at 60°C. The dried product was dried in a drier at 120°C overnight and then calcined in a muffle furnace at 650°C for 3 hours, to obtain a platinum-supporting tin oxide catalyst containing 0.4 wt% of platinum.

1 ml of the catalyst thus prepared, was packed into a quartz tubular reactor having an inner diameter of 6.7 mm. Quartz chips were packed thereon. Then, while permitting hydrogen gas to flow through the tubular reactor at a flow rate of about 50 ml/min, the temperature was raised to 500°C, and then catalytic reduction treatment was carried out at the same temperature for one hour.

### Reaction

Then, hydrogen was switched to nitrogen, and the interior of the reactor was sufficiently substituted by nitrogen, whereupon the gas mixture comprising styrene (hereinafter sometimes referred to as SM), ethylbenzene (hereinafter sometimes referred to as EB), water, hydrogen and air, was introduced into the tubular reactor to initiate the reaction.

The composition of the gas mixture was:
ethylbenzene/styrene/water/hydrogen/oxygen/nitrogen = 1/1/12/1/0.52/1.95 (molar ratio).

Further, the space velocity in the reactor was:
SV=23,900 hr⁻¹ (calculated at 0°C under 1 atm)
LHSV (ethylbenzene+styrene) = 15 hr⁻¹

Upon expiration of 2 hours after initiation of the reaction, the gas at the outlet of the tubular reactor and the liquid trapped in a liquid receptor were analyzed by gas chromatography, whereby the hydrogen conversion was 96.6%, the oxygen conversion was 100%, and the styrene and ethylbenzene combustion rate was 0.18%. Here, the styrene and ethylbenzene combustion rate is the ratio of the molar amount of the styrene and ethylbenzene lost by the combustion reaction to the molar amount of the styrene and ethylbenzene supplied to the reaction zone.

### EXAMPLE 2

The reaction was carried out in the same manner as in Example 1 except that the reaction temperature was changed to 400°C.

The hydrogen conversion was 99.7%, the oxygen conversion was 100%, and the styrene and ethylbenzene combustion rate was 0.10%.

### Example 3

The reaction was carried out in the same manner as in Example 1 except that the reaction temperature was changed to 600°C.

The hydrogen conversion was 87.1%, the oxygen conversion was 100%, and the styrene and ethylbenzene combustion rate was 0.40%.

### EXAMPLE 4

The reaction was carried out in the same manner as in Example 1 using a platinum-supporting tin oxide catalyst containing 0.4 wt% of platinum, prepared in the same manner as in Example 1 except that tin oxide prepared by changing the calcination temperature to 800°C or 1,200°C was employed.

### In the case where the calcination temperature of tin oxide was 800°C

Hydrogen conversion: 94.1%, oxygen conversion: 100%, and styrene and ethylbenzene combustion rate: 0.24%

### In the case where the calcination temperature of tin oxide was 1,200°C

Hydrogen conversion: 92.6%, oxygen conversion: 100%, and styrene and ethylbenzene combustion rate: 0.27%

### EXAMPLE 5

The catalyst was prepared and evaluated in the same manner as in Example 1 except that the amount of platinum supported was changed to 0.2 wt%, and the results are shown in Table 1.

**Table 1**

| Reaction temperature (°C) | Hydrogen conversion (%) | Oxygen conversion (%) | SM^{*1}+EB^{*2} combustion rate (%) |
|---|---|---|---|
| 400 | 99.2 | 97.9 | 0.08 |
| 500 | 96.6 | 100 | 0.18 |
| 600 | 83.4 | 100 | 0.49 |

| | | | |
|---|---|---|---|
| *1 Styrene | | | |
| *2 Ethylbenzene | | | |

### EXAMPLE 6

The catalyst was prepared and evaluated in the same manner as in Example 1 except that the amount of platinum supported was changed to 0.8 wt%, and the results are shown in Table 2.

**Table 2**

| Reaction temperature (°C) | Hydrogen conversion (%) | Oxygen conversion (%) | SM^{*1}+EB^{*2} combustion rate (%) |
|---|---|---|---|
| 400 | 99.7 | 100 | 0.10 |
| 500 | 96.6 | 100 | 0.18 |
| 600 | 87.1 | 100 | 0.40 |

| | | | |
|---|---|---|---|
| *1 Styrene | | | |
| *2 Ethylbenzene | | | |

### COMPARATIVE EXAMPLE 1

The catalyst was prepared and evaluated in the same manner as in Example 1 except that tin oxide in Example 1 was changed to α-Al₂O₃, and the results are shown in Table 3.

**Table 3**

| Reaction temperature (°C) | Hydrogen conversion (%) | Oxygen conversion (%) | SM^{*1}+EB^{*2} combustion rate (%) |
|---|---|---|---|
| 500 | 87.0 | 100 | 0.40 |
| 600 | 67.1 | 100 | 0.88 |

| | | | |
|---|---|---|---|
| *1 Styrene | | | |
| *2 Ethylbenzene | | | |

### EXAMPLE 7

### Preparation of a catalyst

500 ml of titanium isopropoxide was gradually added to 2 ℓ of deionized water cooled by ice water to a temperature of not higher than 5°C with stirring to form a hydroxide. The formed precipitate of titanium hydroxide was collected by filtration, washed with deionized water and then dried in a drier at 120°c overnight. A small amount of deionized water was added to the dried product, and the mixture was kneaded for 3 hours and then extrusion-molded into pellets having a diameter of 3 mm. The molded product was dried in a drier at 120°C overnight and further calcined in a muffle furnace at 1,200°C for 3 hours.

The obtained titanium oxide was pulverized to a particle size of from 0.85 to 1.0 mm, and an aqueous solution of chloroplatinic acid in an amount corresponding to 0.4 wt% was uniformly added thereto, followed by drying under reduced pressure at 60°C by a rotary evaporator. The dried product was further dried in a direr at 120°C overnight and then calcined in a muffle furnace at 650°C for 3 hours to obtain a 0.4 wt% Pt/TiO₂ catalyst.

### Reaction

1 ml of the catalyst thus prepared was packed into a quartz tubular reactor having an inner diameter of about 7 mm and quartz chips having approximately the same particle size as the catalyst were packed on and under the catalyst. Then, while permitting a gas mixture of hydrogen and nitrogen to flow therethrough, reduction treatment was carried out at 500°C for one hour. After the reduction treatment, the temperature of the catalyst layer was changed to a desired temperature under the same atmosphere, and then the interior of the reactor was substituted by nitrogen gas. Then, a gas mixture comprising styrene, ethylbenzene, water, hydrogen and air, was introduced into the tubular reactor, whereupon the reaction and evaluation were carried out in the same manner as in Example 1.

The results are shown in Table 4.

**Table 4**

| Reaction temperature (°C) | Hydrogen conversion (%) | Oxygen conversion (%) | SM^{*1}+EB^{*2} combustion rate (%) |
|---|---|---|---|
| 400 | 97.4 | 100 | 0.16 |
| 500 | 92.3 | 100 | 0.28 |
| 600 | 82.3 | 100 | 0.52 |

| | | | |
|---|---|---|---|
| *1 Styrene | | | |
| *2 Ethylbenzene | | | |

### EXAMPLE 8

### Preparation of a catalyst

A small amount of deionized water was added to 100 g of basic niobium hydroxide (NbO(OH)₃), and the mixture was kneaded for one hour and then extrusion-molded into pellets having a diameter of 3 mm. The molded product was dried in a drier at 120°C overnight and further calcined in a muffle furnace at 1,000°C for 3 hours.

The obtained niobium oxide molded product was pulverized to a particle size of from 0.85 to 1.0 mm, and an aqueous solution of chloroplatinic acid in an amount corresponding to 0.4 wt% of Pt was uniformly added thereto, followed by drying under reduced pressure at 60°C by a rotary evaporator. The dried product was further dried by a drier at 120°C overnight and calcined in a muffle furnace at 650°C for 3 hours to obtain a 0.4 wt% Pt/Nb₂O₅ catalyst.

### Reaction

1 ml of the catalyst thus prepared, was packed into a quartz tubular reactor having an inner diameter of about 7 mm, and quartz chips having approximately the same particle size as the catalyst were packed on and under the catalyst. Then, while permitting a gas mixture of hydrogen and nitrogen to flow therethrough, reduction treatment was carried out at 500°C for one hour. After the reduction treatment, the temperature of the catalyst layer was changed to a desired temperature under the same atmosphere, and then the interior of the reactor was substituted by nitrogen gas. Then, a gas mixture comprising styrene, ethylbenzene, water, hydrogen and air, was introduced into the tubular reactor, whereupon the reaction and evaluation were carried out in the same manner as in Example 1.

The results are shown in Table 5.

**Table 5**

| 0.4 wt% Pt/Nb₂O₅ catalyst | | | |
|---|---|---|---|
| Reaction temperature (°C) | Hydrogen conversion (%) | Oxygen conversion (%) | SM^{*1}+EB^{*2} combustion rate (%) |
| 500 | 97.1 | 98.5 | 0.13 |
| 600 | 86.8 | 100 | 0.41 |

| | | | |
|---|---|---|---|
| *1 Styrene | | | |
| *2 Ethylbenzene | | | |

### EXAMPLE 9

### Preparation of a catalyst

A small amount of deionized water was added to 100 g of tantalum oxide (Ta₂O₅), and the mixture was kneaded for one hour and then extrusion-molded into pellets having a diameter of 3 mm. The molded product was dried in a drier at 120°C overnight and further calcined in a muffle furnace at 1,000°C for 3 hours.

The obtained tantalum oxide molded product was pulverized to a particle size of from 0.85 to 1.0 mm, and an aqueous solution of chloroplatinic acid in an amount corresponding to 0.4 wt% of Pt was uniformly added thereto, followed by drying under reduced pressure at 60°C by a rotary evaporator. The dried product was further dried in a drier at 120°C overnight and then calcined in a muffle furnace at 650°C for 3 hours. to obtain a 0.4 wt% Pt/Ta₂O₅ catalyst.

### Reaction

1 ml of the catalyst thus prepared, was packed into a quartz tubular reactor having an inner diameter of about 7 mm, and quartz chips having approximately the same particle size as the catalyst were packed on and under the catalyst. Then, while permitting a gas mixture of hydrogen and nitrogen to flow therethrough, reduction treatment was carried out at 500°C for one hour. After the reduction treatment, the temperature of the catalyst layer was changed to a desired temperature, and then the interior of the reactor was substituted by nitrogen gas. Then, a gas mixture comprising styrene, ethylbenzene, water, hydrogen and air, was introduced into the tubular reactor, whereupon the reaction and evaluation were carried out in the same manner as in Example 1.

The results are shown in Table 6.

**Table 6**

| 0.4 wt% Pt/Ta₂O₅ catalyst | | | |
|---|---|---|---|
| Reaction temperature (°C) | Hydrogen conversion (%) | Oxygen conversion (%) | SM^{*1}+EB^{*2} combustion rate (%) |
| 400 | 98.4 | 100 | 0.08 |
| 500 | 94.3 | 100 | 0.23 |
| 600 | 83.9 | 100 | 0.47 |

| | | | |
|---|---|---|---|
| *1 Styrene | | | |
| *2 Ethylbenzene | | | |

### EXAMPLE 10

### Preparation of a catalyst

3N aqueous ammonia was gradually added to an aqueous stannous chloride solution with stirring to form a precipitate of a hydroxide. When the pH exceeded 7, addition of aqueous ammonia was stopped, and the formed precipitate of tin hydroxide was collected by filtration, washed with deionized water and then dried in a drier at 120°C overnight. The dried precipitate was calcined in a muffle furnace at 600°C for 3 hours.

A small amount of deionized water was added to the calcined product, and the mixture was kneaded for one hour and extrusion-molded into pellets having a diameter of 3 mm. The molded product was dried in a drier at 120°C overnight and further calcined in a muffle furnace at 1,000°C for 3 hours.

The obtained tin oxide was pulverized to a particle size of from 0.85 to 1.0 mm, and a hydrochloric acid aqueous solution of palladium chloride in an amount corresponding to 0.4 wt% of palladium, was uniformly added thereto, followed by drying under reduced pressure at 60°C by a rotary evaporator. The dried product was further dried in a drier at 120°C overnight and then calcined in a muffle furnace at 550°C for 3 hours to obtain a 0.4 wt% Pd/SnO₂ catalyst.

### Reaction

1 ml of the catalyst thus prepared, was packed into a quartz tubular reactor having an inner diameter of about 7 mm, and quartz chips having approximately the same particle size as the catalyst were packed on and under the catalyst. Then, while permitting a gas mixture of hydrogen and nitrogen to flow therethrough, reduction treatment was carried out at 500°C for one hour. After the reduction treatment, the temperature of the catalyst layer was changed to a desired temperature, and the interior of the reactor was substituted by nitrogen gas. Then, a gas mixture comprising styrene, ethylbenzene, water, hydrogen and air, was introduced into the tubular reactor, whereupon the reaction and evaluation were carried out in the same manner as in Example 1.

The results are shown in Table 7.

**Table 7**

| 0.4 wt% Pd/SnO₂ catalyst | | | |
|---|---|---|---|
| Reaction temperature (°C) | Hydrogen conversion (%) | Oxygen conversion (%) | SM^{*1}+EB^{*2} combustion rate (%) |
| 400 | 96.1 | 100 | 0.18 |
| 500 | 87.9 | 100 | 0.38 |
| 600 | 78.3 | 100 | 0.61 |

| | | | |
|---|---|---|---|
| *1 Styrene | | | |
| *2 Ethylbenzene | | | |

### EXAMPLE 11

The catalyst was prepared and evaluated in the same manner as in Example 10 except that the amount of palladium supported, was changed to 1.0 wt%, and the results are shown in Table 8.

**Table 8**

| 1.0 wt% Pd/SnO₂ catalyst | | | |
|---|---|---|---|
| Reaction temperature (°C) | Hydrogen conversion (%) | Oxygen conversion (%) | SM^{*1}+EB^{*2} combustion rate (%) |
| 400 | 95.8 | 100 | 0.20 |
| 500 | 88.0 | 100 | 0.38 |
| 600 | 81.5 | 100 | 0.54 |

| | | | |
|---|---|---|---|
| *1 Styrene | | | |
| *2 Ethylbenzene | | | |

As shown by the foregoing Examples, by the method of the present invention, hydrogen can selectively be oxidized, and the loss due to combustion of a co-existing hydrocarbon, can be suppressed to such a sufficiently low level that there will be substantially no problem.

## Claims

1. A method for selective oxidation of hydrogen, which comprises selectively oxidizing hydrogen in a gas mixture containing a hydrocarbon and hydrogen by contacting the gas mixture with an oxygen-containing gas in the presence of an oxidation catalyst, wherein a catalyst comprising a component having platinum and/or palladium supported on at least one carrier calcinated at a temperature of from 800 to 1500°C and selected from the group consisting of tin oxide, titanium oxide, tantalum oxide and niobium oxide, is used as the oxidation catalyst.

2. The method according to claim 1, wherein a catalyst comprising a component having platinum supported on tin oxide, titanium oxide, tantalum oxide or niobium oxide, or a catalyst comprising a component having palladium supported on tin oxide, is used as the oxidation catalyst.

3. The method according to claim 1 or 2, wherein the gas mixture containing a hydrocarbon and hydrogen is a gas mixture which is obtained by subjecting a feed hydrocarbon to a dehydrogenation reaction in the presence of a dehydrogenation catalyst and which comprises a dehydrogenated hydrocarbon, an unreacted feed hydrocarbon and hydrogen.

4. The method according to claim 1, 2 or 3, wherein the hydrocarbon is a mixture of ethylbenzene and styrene.

5. The method according to claim 1, 2, 3 or 4, wherein the gas mixture containing a hydrocarbon and hydrogen, is contacted with the oxygen-containing gas within a temperature range of from 300 to 800°C.

6. The method according to any one of claims 1 to 5, wherein the amount of platinum and/or palladium supported, is from 0.01 to 10 wt%, based on the carrier.

7. A method for dehydrogenation of a hydrocarbon, which comprises selectively oxidizing hydrogen in a gas mixture which is obtained by subjecting a feed hydrocarbon to a dehydrogenation reaction in the presence of a dehydrogenation catalyst and which comprises a dehydrogenated hydrocarbon, an unreacted feed hydrocarbon and hydrogen, by contacting the gas mixture with an oxygen-containing gas in the presence of an oxidation catalyst, and further subjecting a hydrocarbon-containing gas obtained by the oxidation raction to a dehydrogenation reaction, wherein a catalyst comprising component having platinum or palladium supported on at least one carrier calcined at a temperature of from 800 to 1500°C and selected from the group consisting of tin oxide, titanium oxide, tantalum oxide and niobium oxide, is used as the oxidation catalyst.

8. The method according to claim 7, wherein a catalyst comprising a component having platinum supported on tin oxide, titanium oxide, tantalum oxide or niobium oxide, or a catalyst comprising a component having palladium supported on tin oxide, is used as the oxidation catalyst.

9. The method according to claim 7 or 8, wherein the feed hydrocarbon is ethylbenzene, and the dehydrogenated hydrocarbon is styrene.

## Patentansprüche

1. Verfahren zur selektiven Oxidation von Wasserstoff, umfassend das selektive Oxidieren von Wasserstoff in einer einen Kohlenwasserstoff und Wasserstoff enthaltenden Gasmischung durch Kontaktieren der Gasmischung mit einem sauerstoffhaltigen Gas in Gegenwart eines Oxidationskatalysators, wobei als Oxidationskatalysator ein Katalysator verwendet wird, welcher eine Komponente umfaßt, bei der Platin und/oder Palladium auf mindestens einem Träger getragen wird, der bei einer Temperatur von 800 bis 1500°C kalziniert worden ist und aus der Zinnoxid, Titanoxid, Tantaloxid und Nioboxid umfassenden Gruppe gewählt ist.

2. Verfahren nach Anspruch 1, wobei ein Katalysator, umfassend eine Komponente, bei der Platin auf Zinnoxid, Titanoxid, Tantaloxid oder Nioboxid getragen wird, oder ein Katalysator, umfassend eine Komponente, bei der Palladium auf Zinnoxid getragen wird, als Oxidationskatalysator verwendet wird.

3. Verfahren nach Anspruch 1 oder 2, wobei die einen Kohlenwasserstoff und Wasserstoff enthaltende Gasmischung eine Gasmischung ist, die dadurch erhalten wird, daß ein Einspeisungs-Kohlenwasserstoff einer Dehydrierungsreaktion in Gegenwart eines Dehydrierungskatalysators unterzogen wird, und welche einen dehydrierten Kohlenwasserstoff, einen nicht umgesetzten Einspeisungs-Kohlenwasserstoff und Wasserstoff enthält.

4. Verfahren nach Anspruch 1, 2 oder 3, wobei der Kohlenwasserstoff eine Mischung aus Ethylbenzol und Styrol ist.

5. Verfahren nach Anspruch 1, 2, 3 oder 4, wobei die einen Kohlenwasserstoff und Wasserstoff enthaltende Gasmischung mit dem sauerstoffhaltigen Gas innerhalb eines Temperaturbereiches von 300 bis 800°C kontaktiert wird.

6. Verfahren nach mindestens einem der Ansprüche 1 bis 5, wobei die Menge an getragenem Platin und/oder Palladium 0,01 bis 10 Gew.-% beträgt, bezogen auf den Träger.

7. Verfahren zur Dehydrierung eines Kohlenwasserstoffs, umfassend das selektive Oxidieren von Wasserstoff in einer Gasmischung, welche erhalten wird durch Unterziehen eines Einspeisungs-Kohlenwasserstoffs einer Dehydrierungsreaktion in Gegenwart eines Dehydrierungskatalysators, und welche einen dehydrierten Kohlenwasserstoff, einen nicht umgesetzten Einspeisungs-Kohlenwasserstoff und Wasserstoff umfaßt, durch Kontaktieren der Gasmischung mit einem sauerstoffhaltigen Gas in Gegenwart eines Oxidationskatalysators, und weiterhin Unterziehen eines durch die Oxidationsreaktion erhaltenen, einen Kohlenwasserstoff enthaltenen Gases einer Dehydrierungsreaktion, wobei ein Katalysator, umfassend eine Komponente, bei der Platin oder Palladium auf mindestens einem Träger getragen wird, der bei einer Temperatur von 800 bis 1500°C kalziniert worden ist und aus der Zinnoxid, Titanoxid, Tantaloxid und Nioboxid umfassenden Gruppe gewählt ist, als Oxidiationskatalysator verwendet wird.

8. Verfahren nach Anspruch 7, wobei ein Katalysator, umfassend eine Komponente, bei der Platin auf Zinnoxid, Titanoxid, Tantaloxid oder Nioboxid getragen wird, oder ein Katalysator, umfassend eine Komponente, bei der Palladium auf Zinnoxid getragen wird, als Oxidationskatalysator verwendet wird.

9. Verfahren nach Anspruch 7 oder 8, wobei der Einspeisungs-Kohlenwasserstoff Ethylbenzol ist, und der dehydrierte Kohlenwasserstoff Styrol ist.

## Revendications

1. Procédé pour l'oxydation sélective d'hydrogène, qui comprend le fait d'oxyder sélectivement de l'hydrogène dans un mélange gazeux contenant un hydrocarbure et de l'hydrogène en mettant en contact le mélange gazeux avec un gaz contenant de l'oxygène en présence d'un catalyseur d'oxydation, dans lequel est utilisé en tant que catalyseur d'oxydation, un catalyseur comprenant un constituant à base de platine et/ou de palladium supporté sur au moins un support calciné à une température de 800°C à 1500°C et choisi dans le groupe constitué de l'oxyde d'étain, de l'oxyde de titane, de l'oxyde de tantale et de l'oxyde de niobium.

2. Procédé selon la revendication 1, dans lequel est utilisé en tant que catalyseur d'oxydation, un catalyseur comprenant un constituant à base de platine supporté sur de l'oxyde d'étain, de l'oxyde de titane, de l'oxyde de tantale ou de l'oxyde de nobium, ou un catalyseur comprenant un constituant à base de palladium supporté sur de l'oxyde d'étain.

3. Procédé selon la revendication 1 ou 2, dans lequel le mélange gazeux contenant un hydrocarbure et de l'hydrogène est un mélange gazeux qui est obtenu en soumettant un hydrocarbure d'alimentation à une réaction de déhydrogénation en présence d'un catalyseur de déhydrogénation et qui comprend un hydrocarbure déhydrogéné, un hydrocarbure d'alimentation non réagi et de l'hydrogène.

4. Procédé selon la revendication 1, 2 ou 3, dans lequel l'hydrocarbure est un mélange d'éthylbenzène et de styrène.

5. Procédé selon la revendication 1, 2, 3 ou 4, dans lequel le mélange gazeux contenant un hydrocarbure et de l'hydrogène est mis en contact avec un gaz contenant de l'hydrogène dans un intervalle de température allant de 300°C à 800°C.

6. Procédé selon l'une quelconque des revendications 1 à 5, dans lequel la quantité de platine et/ou de palladium supportée est de 0,01 à 10 % en poids sur la base du support.

7. Procédé pour la déhydrogénation d'un hydrocarbure qui comprend le fait d'oxyder sélectivement de l'hydrogène dans un mélange gazeux qui est obtenu en soumettant un hydrocarbure d'alimentation à une réaction de déhydrogénation en présence d'un catalyseur de déhydrogénation et qui comprend un hydrocarbure déhydrogéné, un hydrocarbure d'alimentation non réagi et de l'hydrogène, par mise en contact du mélange gazeux avec un gaz contenant de l'oxygène en présence d'un catalyseur d'oxydation, et en soumettant en outre le gaz contenant un hydrocarbure obtenu par la réaction d'oxydation à une réaction de déhydrogénation, dans lequel est utilisé en tant que catalyseur d'oxydation un catalyseur comprenant un constituant à base de platine ou de palladium supporté sur au moins un support calciné à une température de 800°C à 1500°C et choisi dans le groupe constitué de l'oxyde d'étain, de l'oxyde de titane, de l'oxyde de tantale et de l'oxyde de niobium.

8. Procédé selon la revendication 7, dans lequel est utilisé en tant que catalyseur d'oxydation, un catalyseur comprenant un constituant à base de platine supporté sur de l'oxyde d'étain, de l'oxyde de titane, de l'oxyde de tantale ou de l'oxyde de nobium, ou un catalyseur comprenant un constituant à base de palladium supporté sur de l'oxyde d'étain.

9. Procédé selon la revendication 7 ou 8, dans lequel l'hydrocarbure d'alimentation est de l'éthylbenzène et l'hydrocarbure déhydrogéné est le styrène.
